# FASCICULE DE BREVET EUROPEEN

(11) **EP 2 263 613 B1**
(45) Date de publication et mention de la délivrance du brevet: **19.12.2012**
(21) Numéro de dépôt: 10166096.7
(22) Date de dépôt: 16.06.2010
(51) Int. Cl.: A61F 2/40

(54) **Composant prothétique glénoïdien et jeu d'au moins deux de ces composants**
Prothetische Glenoidkomponente und Satz von mindestens zwei dieser Komponenten
Prosthetic glenoid component and set of at least two of these components

(30) Priorité: 17.06.2009 FR 0954078
(43) Date de publication de la demande: 22.12.2010
(73) Titulaire: Tornier, 38330 Montbonnot-Saint-Martin (FR)
(72) Inventeur: Brunnarius, Yann, 26300, CHATUZANGE LE GOUBET (FR)
(74) Mandataire: Grand, Guillaume

(56) Documents cités:
- EP-A- 1 776 935
- EP-A- 1 844 737
- WO-A-2007/109319
- US-A- 5 358 525

## Description

La présente invention concerne un composant prothétique glénoïdien, ainsi qu'un jeu de tels composants.

L'invention s'intéresse plus particulièrement à la restauration articulaire anatomique de la glène de l'omoplate d'un être humain, par un composant prothétique définissant une surface concave sur laquelle vient d'appuyer et s'articuler une tête humérale convexe soit prothétique, soit osseuse.

Un grand nombre des composants prothétiques glénoïdiens actuels donnent satisfaction en ce qui concerne leur comportement cinématique pour l'articulation de l'épaule des patients. Toutefois, on constate que, en service, ces composants glénoïdiens induisent très fréquemment des surtensions sur l'environnement musculo-ligamentaire de l'épaule, sont rapidement sujets à des usures et des descellements, et/ou rendent instable l'articulation de l'épaule. Plus généralement, on peut dire que les performances cinématiques des composants glénoïdiens connus sont atteintes lorsque la tête humérale glisse, tourne et reste stable sur le composant glénoïdien. Par ailleurs, ces performances sont amoindries lorsqu'un des paramètres cinématiques au moins (rotation, glissement, stabilité) est dégradé.

A l'inverse, EP-A-1 776 935 a proposé de revêtir un corps prothétique suffisamment rigide pour être solidarisé fermement à l'os de la glène, par un corps souple d'appui pour la tête humérale. Ce corps d'appui souple est réalisé en un matériau élastique, tel que du polyuréthane élastomérique, qui forme, tout autour de sa partie centrale contre laquelle s'articule la tête humérale, un bord périphérique déformable dans son épaisseur, pour simuler un bourrelet marginal anatomique, parfois désigné sous son terme latin « labrum ». L'effet de ce bord déformable présente un réel intérêt dans le sens où il induit une coopération, avec la tête humérale, plus proche d'un comportement anatomique naturel. Toutefois, le matériau élastique précité, utilisé pour réaliser le corps d'appui souple, ne permet pas, en service, de garantir ni des performances cinématiques satisfaisantes pour l'articulation de l'épaule, ni une résistance suffisante à l'usure.

Le but de la présente invention est de proposer un composant glénoïdien qui, lorsqu'il coopère avec une tête humérale, concilie de bonnes performances cinématiques et un comportement le plus naturel possible.

A cet effet, l'invention a pour objet un composant prothétique glénoïdien, tel que défini à la revendication 1.

L'idée à la base de l'invention est de chercher à simuler un bourrelet marginal glénoïdien anatomique par le bord périphérique du corps d'appui, tout en réalisant ce bord périphérique avec un matériau aussi rigide, voire le même matériau, que celui utilisé pour réaliser la partie principale de ce corps d'appui. Bien entendu, la partie principale et le bord périphérique sont alors avantageusement réalisés d'un seul tenant, en étant venus de matière l'un avec l'autre. Pour ce faire, l'invention prévoit d'affaiblir mécaniquement la zone de liaison entre la partie principale et le bord périphérique, de manière à rendre cette zone de liaison élastiquement déformable par rapport au reste du corps d'appui. L'affaiblissement mécanique est conçu pour que, sous l'action de la tête humérale sur le bord périphérique, la zone de liaison précitée se déforme, en conférant alors au bord périphérique un comportement similaire, voire identique à celui d'un bourrelet marginal anatomique glénoïdien : ce bord périphérique permet ainsi de freiner, retenir et recentrer la tête humérale lorsque, en service, elle s'écarte de la région centrale de la partie principale du corps d'appui jusqu'à s'appuyer contre le bord périphérique. Dans le même temps, cette partie centrale peut présenter une géométrie et une dureté qui garantissent des performances cinématiques élevées et pérennes pour l'articulation entre le corps d'appui et la tête humérale.

D'autres caractéristiques avantageuses du composant prothétique glénoïdien conforme à l'invention, prises isolément ou selon toutes les combinaisons techniquement possibles, sont spécifiées aux revendications dépendantes 2 à 9.

L'invention a également pour objet un jeu d'au moins deux composants prothétiques glénoïdiens, tel que défini à la revendication 10.

Grâce au jeu conforme à l'invention, un chirurgien dispose de plusieurs composants glénoïdiens contre lesquels une même tête humérale pourra s'articuler de manière sensiblement identique. En revanche, selon le composant glénoïdien choisi par le chirurgien parmi ceux à sa disposition, l'effet du bord périphérique du corps d'appui de ce composant, sur la tête humérale, sera légèrement différent : en effet, l'amplitude de la déformation élastique de la zone de liaison entre le bord périphérique et la partie principale de ce corps d'appui sera influencée par son positionnement relatif vis-à-vis de l'os de la glène dans lequel le composant sera implanté, en particulier selon que cette zone de liaison se retrouve située essentiellement à l'aplomb du pourtour périphérique de la glène ou bien à l'extérieur ou à l'intérieur de ce pourtour périphérique.

L'invention sera mieux comprise à la lecture de la description qui va suivre, donnée uniquement à titre d'exemple et faite en se référant aux dessins sur lesquels :
- la figure 1 est une représentation schématique de principe d'une prothèse d'épaule implantée sur un patient, comprenant un composant glénoïdien conforme à l'invention ;
- la figure 2 est une vue en perspective montrant seul le composant glénoïdien de la figure 1 ;
- la figure 3 est une vue en élévation selon la flèche III de la figure 2 ;
- les figures 4 et 5 sont des coupes selon respectivement les lignes IV-IV et V-V de la figure 3 ;
- la figure 6 est une vue à plus grande échelle du détail cerclé VI sur la figure 4 ;
- la figure 7 est une vue analogue à la figure 5, montrant, de manière schématique, le composant glénoïdien des figures 1 à 6 ainsi que deux autres composants glénoïdiens, ces trois composants appartenant à un jeu conforme à l'invention ; et
- les figures 8 à 12 illustrent un autre mode de réalisation d'un composant glénoïdien conforme à l'invention, ces figures étant respectivement analogues aux figures 2 à 6.

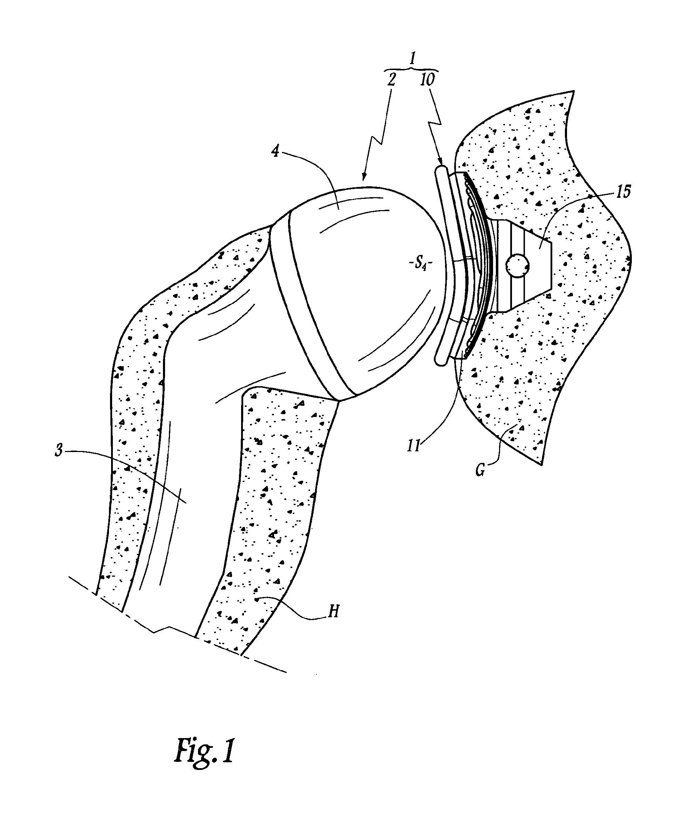

Sur la figure 1 est représentée une prothèse d'épaule totale 1 comprenant un composant 10 fixé à la glène G de l'omoplate d'un patient. Ce composant glénoïdien 10 est adapté pour coopérer par contact articulé avec un composant 2 de la prothèse 1, fixé à l'humérus H associé à l'épaule du patient. Dans l'exemple de réalisation considéré sur la figure, ce composant huméral 2 comporte une tige 3, à même d'être ancrée fixement dans le canal médullaire de l'humérus H, et une tête 4, portée fixement par la tige et délimitant extérieurement une surface convexe S₄, ici de forme globalement hémisphérique.

Lorsque la prothèse 1 est en service, la surface convexe S₄ de la tête humérale 4 s'appuie et s'articule contre le composant glénoïdien 10, comme expliqué en détail ci-après. A titre de variante non représentée, plutôt que le composant glénoïdien 10 coopère en service avec une tête humérale prothétique telle que la tête 4, la tête osseuse de l'humérus H peut être appuyée de manière articulée contre ce composant 10 : dans ce cas, la prothèse d'épaule n'inclut plus de composant huméral et est généralement qualifiée de prothèse partielle.

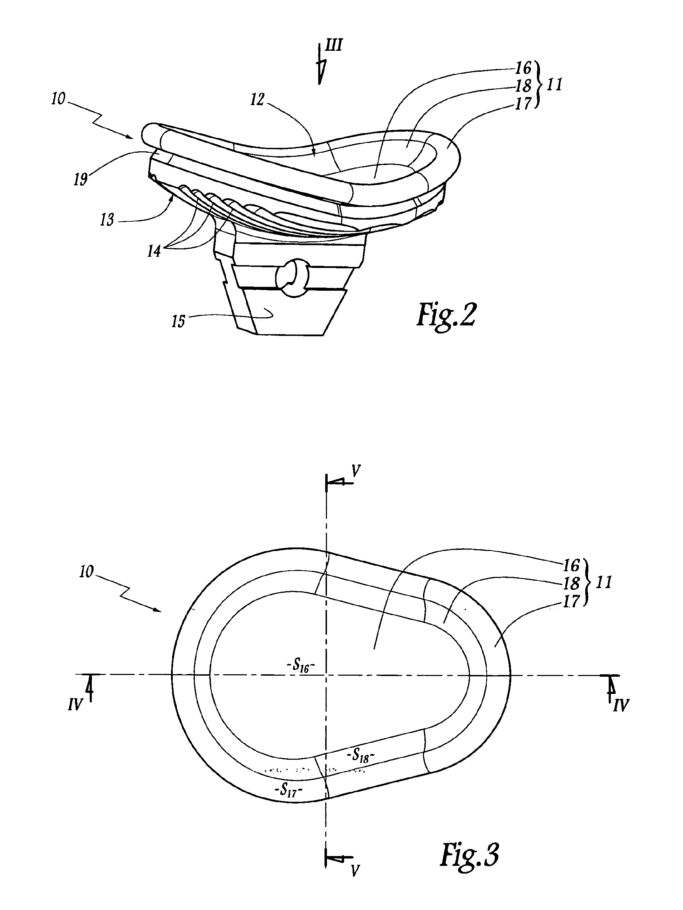

Comme montré plus en détail sur les figures 2 à 6, le composant glénoïdien 10 comporte un corps monobloc 11 en forme globale de coupelle. Le corps 11 présente ainsi deux faces principales opposées, à savoir une face 12 essentiellement en creux, destinée à être tournée vers la tête humérale 4, et une face 13 globalement bombée ou plate, destinée à être plaquée et solidarisée contre l'os de la glène G.

En pratique, les aménagements de la face 13 afin de fixer le corps 11 à la glène G ne sont pas limitatifs de l'invention. Ainsi, dans l'exemple de réalisation considéré sur les figures, d'une part, des sillons 14, adaptés pour venir en prise directe avec la surface osseuse de la glène G, sont délimités dans la face 13 et, d'autre part, la région centrale de cette face 13 est munie, ici de façon monobloc, d'une quille saillante 15 dimensionnée pour venir s'ancrer profondément dans la glène G. Avantageusement, cette quille 15 est traversée par un trou à l'intérieur duquel du ciment peut être injecté, afin de verrouiller et de stabiliser la face 13 par rapport à la glène. L'immobilisation de la quille peut par ailleurs être renforcée par une vis transversale. A titre de variantes non représentées, les aménagements de la face 13 peuvent comprendre ou consister en plusieurs plots saillants d'emmanchement dans la matière osseuse de la glène, en un ou plusieurs trous traversants dans lesquels sont reçus des vis ou, plus généralement, des organes d'ancrage osseux avec la glène, et/ou en un revêtement à base d'hydroxyapatite pour faciliter l'ostéo-intégration, etc.

Comme bien visible sur les figures 3 à 5, le corps 11 inclut, à la fois, une partie principale 16, qui, du côté de la face 13, porte les sillons 14 et l'aile 15, un bord périphérique 17, qui s'étend tout autour de la partie principale 16, et une zone 18 reliant l'un à l'autre la partie principale 16 et le bord périphérique 17.

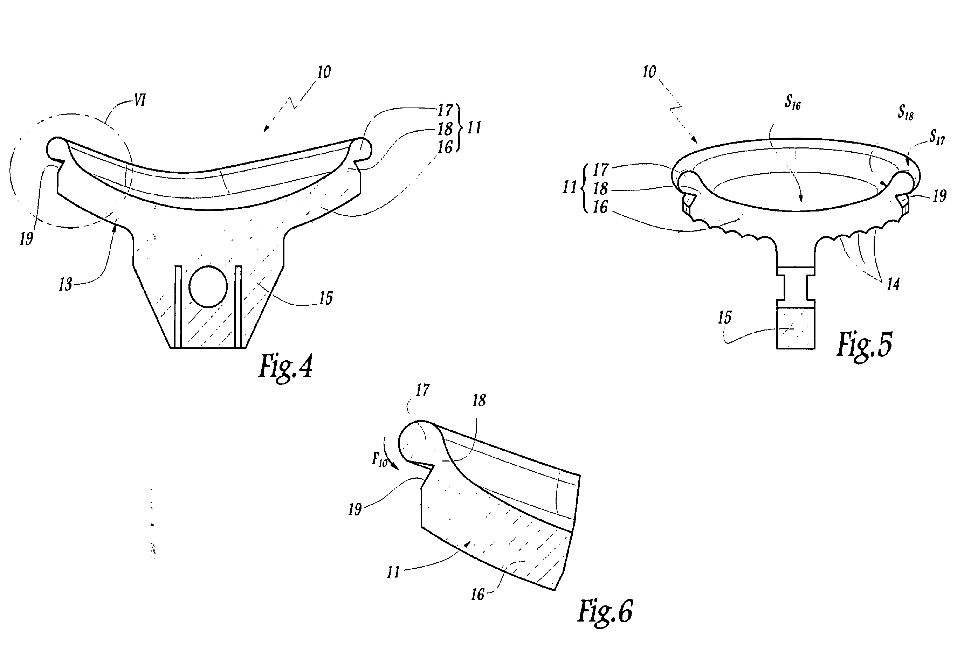

Le bord périphérique 17 présente, sur la face 12 du corps d'appui 11, une surface convexe S₁₇ qui, en coupe transversale comme sur les figures 4 et 5, présente un profil incurvé. Dans l'exemple de réalisation considéré sur les figures, ce profil incurvé correspond sensiblement à un arc de cercle.

La partie principale 16 et la zone de liaison 18 présentent, quant à elles, sur la face 12 du corps 11, des surfaces concaves, respectivement référencées S₁₆ et S₁₈. La surface concave S₁₆ est dimensionnée pour coopérer de façon articulaire, c'est-à-dire en rotation et glissement, avec la surface convexe S₄ de la tête humérale 4. De façon connue en soi, les géométries respectives de ces surfaces S₄ et S₁₆ sont conçues pour reproduire aussi fidèlement que possible les comportements articulaires anatomiques de l'épaule.

Avantageusement, pour des raisons qui apparaitront plus loin, la courbure de la surface S₁₈ est plus grande que celle de la surface S₁₆, comme bien visible en coupe sur les figures 4 et 5.

Avantageusement, en observant le corps 11 suivant une direction normale à sa face 12, comme sur la figure 3, les contours périphériques respectifs de la partie principale 16, de la zone de liaison 18 et du bord périphérique 17 ont une forme reproduisant celle du contour périphérique de la glène. En pratique, cette forme est ovale, voire avantageusement s'apparente à un profil en poire, c'est-à-dire à un profil allongé aux extrémités opposées arrondies, avec l'une de ces extrémités présentant un rayon de courbure plus important que l'autre extrémité. Cette forme particulière reproduit fidèlement l'anatomie correspondante de la glène humaine.

Comme bien visible sur la figure 6, la zone de liaison 18 est munie d'une gorge périphérique 19, délimitée dans la face 13 du corps 11. Cette gorge 19 forme ainsi un creux dans la matière constituant la zone de liaison 18, réduisant l'épaisseur de cette dernière par rapport, d'une part, à l'épaisseur de la partie principale 16 et, d'autre part, à l'épaisseur du bord périphérique 17.

Dans l'exemple de réalisation considéré sur les figures, la gorge 19 s'étend de manière continue sur toute la périphérie du corps 11, sans interruption. En variante non représentée, cette gorge 19 pourrait être interrompue en une ou plusieurs portions suivant la périphérie du corps 11.

De même, dans le mode de réalisation considéré sur les figures, la gorge 19 présente, en coupe transversale, une section à profil en V. Cette forme de profil n'est pas limitative de l'invention, dans le sens où d'autres formes sont envisageables, telles qu'un profil en U, un profil en W, un profil en « soufflet d'accordéon », etc.

Dans tous les cas, la gorge 19 est conçue et dimensionnée pour affaiblir mécaniquement la zone de liaison 18 afin d'autoriser sa déformation élastique par rapport, à la fois, à la partie principale 16 et au bord périphérique 17. En pratique, cette déformation est réalisée par rapprochement ou écartement des bords en regard de la gorge 19.

En service, c'est-à-dire lorsque la prothèse 1 est implantée et que le patient ainsi prothésé sollicite l'articulation de son épaule, la tête humérale 4 s'appuie contre le corps 11, au niveau de sa face 12. Tant que la tête humérale 4 coopère avec la région centrale de la partie principale 16, les surfaces S₄ et S₁₆ coopèrent de manière articulée, comme expliqué plus haut.

Dans certaines configurations de sollicitation, la tête humérale 4 s'écarte de la région centrale précitée et se rapproche ainsi d'une portion périphérique de la zone de liaison 18 et du bord périphérique 17. Lorsque la surface convexe S₄ de la tête humérale s'appuie sur la surface S₁₈ de la zone de liaison 18, la concavité plus courbée de cette dernière, par rapport à la surface S₁₆, tend à ramener la tête humérale vers la région centrale de la partie principale 16. De plus, lorsque, le cas échéant, la tête humérale 4 s'appuie contre le bord périphérique 17, la surface convexe S₁₇ de ce dernier simule la présence d'un bourrelet marginal glénoïdien anatomique, qui tend ainsi à retenir par butée la tête humérale. De surcroît, l'appui de la tête humérale contre le bord périphérique 17 génère un couple de basculement de ce bord par rapport à la partie principale 16, ce couple tendant à rapprocher l'un de l'autre les bords de la gorge 19, comme indiqué par la flèche F₁₀ sur la figure 6. Il en résulte que la zone de liaison 18 se déforme, si bien que, dès que l'appui de la tête humérale 4 se relâche, l'énergie de déformation accumulée dans cette zone 18 se libère de manière élastique : les bords de la gorge 19 s'écartent, pour reprendre leur configuration initiale de manière que le bord périphérique 17 repousse la tête humérale 4 vers la région centrale de la partie principale 16.

Ainsi, on comprend que la présence de la gorge 19 confère au bord périphérique 17 une capacité de retenue élastique de la tête humérale 4 alors que ce bord périphérique 17 est avantageusement réalisé avec le même matériau dur que le matériau constituant la partie principale 16. En effet, pour garantir la précision et la pérennité de la coopération mécanique articulaire entre la partie principale 16 et la tête humérale 4, cette partie principale 16 est dure, c'est-à-dire réalisée en un matériau qui ne va ni se déformer dans sa masse, ni s'user sous l'action des contraintes couramment constatées au sein d'une articulation d'épaule. Typiquement, le matériau utilisé pour réaliser la partie principale 16 et, plus généralement, pour réaliser tout le corps 11, y compris le bord périphérique 17 et la zone de liaison 18, est choisi parmi du polyéthylène haute densité, un alliage métallique, du pyrocarbone, du polyétheréthercétone (PEEK), etc.

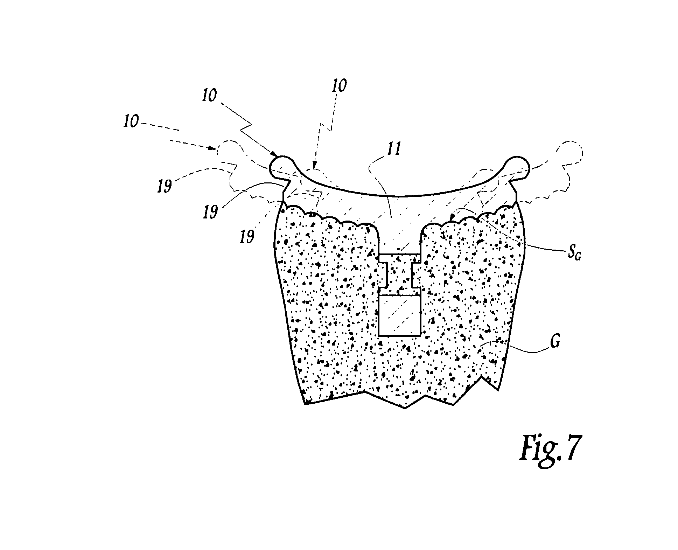

Avantageusement, lors du traitement chirurgical de l'épaule du patient, le chirurgien dispose du composant glénoïdien 10 en différentes tailles. Par exemple, le chirurgien dispose de ce composant glénoïdien 2 en trois tailles différentes, comme montré sur la figure 7 sur laquelle, en plus du composant glénoïdien 10 considéré sur les figures précédentes, qui est représenté en traits pleins, deux autres composants 10, respectivement plus petit et plus grand que le composant 10 des figures précédentes, sont représentés en pointillés. Plus précisément, les trois composants glénoïdiens 10 considérés sur la figure 7 présentent des corps 11 homothétiques, excepté pour ce qui concerne les aménagements de leur face 13, permettant de les fixer à la glène G. Ainsi, les trois composants glénoïdiens 10 présentent des valeurs respectives différentes pour leur dimension suivant n'importe quelle direction passant par deux portions de leur gorge 19, diamétralement opposées par rapport à la région centrale de leur partie principale 16.

Il en résulte que, vis-à-vis de la glène G du patient opéré, le choix du composant glénoïdien 10 parmi le jeu des trois composants disponibles induit un positionnement relatif spécifique pour la gorge 19 : comme montré de manière schématique sur la figure 7, l'implantation du plus petit des trois composants disponibles 10 conduit à positionner la gorge 19 à l'intérieur de la surface osseuse S_{G} de la glène G, tandis que l'implantation du plus grand des trois composants 10 conduit à positionner la gorge 19 à l'extérieur de cette surface osseuse S_{G}. De façon intermédiaire, l'implantation du dernier des trois composants disponibles conduit à positionner la gorge 19 sensiblement à l'aplomb du pourtour périphérique de la surface osseuse S_{G}.

Le comportement mécanique de la zone de liaison 18 est influencé par ce positionnement relatif de la gorge 19 et de la surface osseuse S_{G} de la glène G : selon que la gorge se trouve à l'extérieur, à l'intérieur ou à l'aplomb du pourtour périphérique de cette surface osseuse S_{G}, les bords de la gorge 19 s'écartent l'un de l'autre avec plus ou moins de facilité, c'est-à-dire sous l'action d'un effort plus ou moins important appliqué sur le bord périphérique 17 par la tête humérale 4.

Ainsi, notamment selon les cas pathologiques de reconstruction de l'articulation de l'épaule, le chirurgien peut choisir la taille du composant glénoïdien 10 qu'il considère comme étant la mieux adaptée.

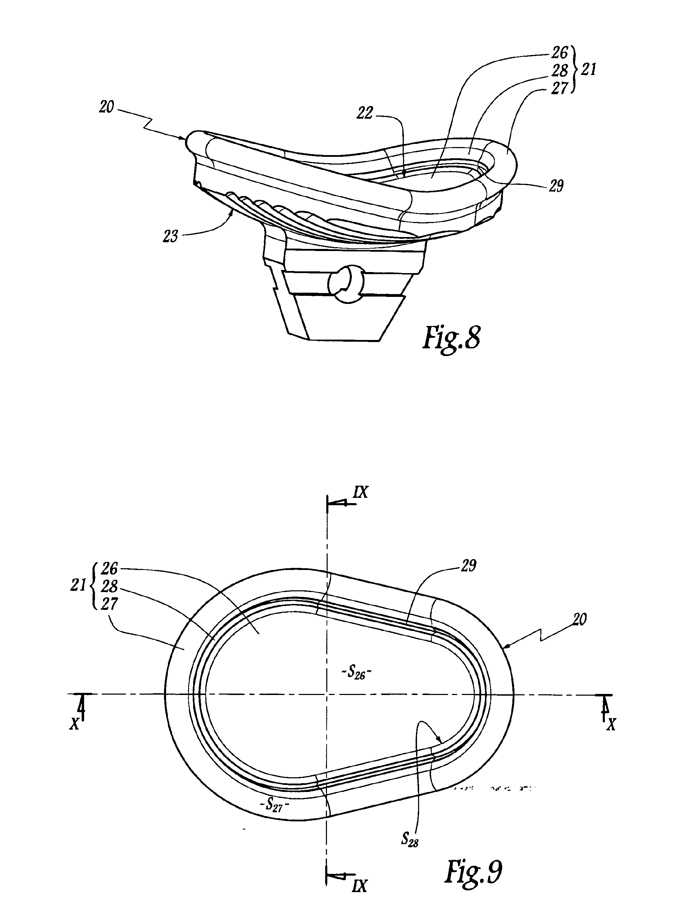

Sur les figures 8 à 12 est représenté un second mode de réalisation d'un composant glénoïdien 20 dont les constituants identiques à ceux du composant glénoïdien 10 sont repérés par les mêmes références numériques, augmentées de 10. Ainsi, le composant 20 comprend un corps 21 d'appui pour la tête humérale 4, ce corps 21 incluant, à la fois, une partie principale 26 d'articulation avec la tête 4, un bord périphérique 27 de simulation d'un bourrelet marginal glénoïdien anatomique, et une zone 28 de liaison entre cette partie principale et ce bord périphérique. Sur la face 22 du corps 21, tournée vers la tête humérale 4 en service, la partie principale 26, le bord périphérique 27 et la zone de liaison 28 présentent des surfaces respectivement concave S₂₆, convexe S₂₇ et concave S₂₈, géométriquement et fonctionnellement analogues aux surfaces S₁₆, S₁₇ et S₁₈ du composant 10. La face opposée 23 du corps 21 est, comme la face 13 du corps 11, adaptée pour être plaquée contre et solidarisée à l'os de la glène G.

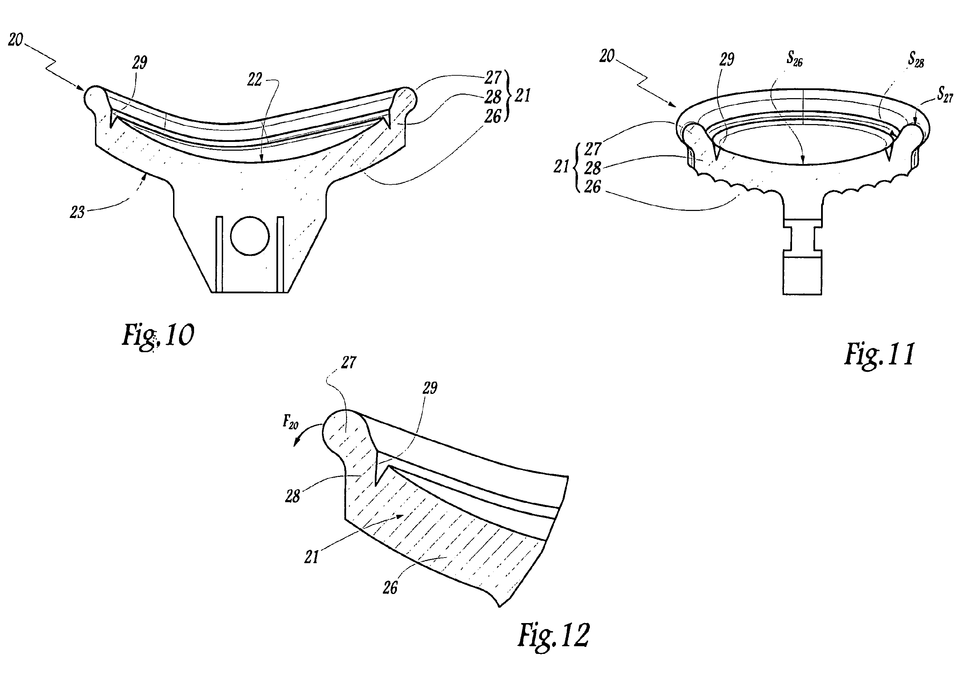

Comme bien visible sur les figures 10 à 12, le composant 20 se distingue du composant 10 par l'aménagement de sa zone de liaison 28, visant à affaiblir mécaniquement cette dernière. En effet, la gorge 19 du composant 10 est remplacée par un sillon 29 délimité dans la face 22 du corps 21.

En service, ce sillon 29 permet, de la même façon que la gorge 19, la déformation élastique de la zone de liaison 28 lorsque la tête humérale 4 s'appuie sur le bord périphérique 27, par écartement et rapprochement relatif des bords en regard de ce sillon 29. Plus précisément, comme indiqué par la flèche F₂₀ sur la figure 12, l'action d'appui de la tête humérale 4 tend à écarter les bords du sillon 29, puis, par rappel élastique de la matière constituant la zone de liaison 28, les bords précités tendent à se rapprocher l'un de l'autre.

Divers aménagements et variantes aux composants glénoïdiens 10 et 20 décrits jusqu'ici sont par ailleurs envisageables. A titre d'exemples :
- la zone de liaison 18 peut inclure plusieurs gorges 19, disposées l'une après l'autre suivant une direction reliant la partie principale 16 et le bord périphérique 17 ; de même, la zone de liaison 28 peut présenter ainsi plusieurs sillons 29 ; et/ou
- les modes de réalisation des composants 10 et 20 peuvent être combinés, c'est-à-dire que la zone de liaison entre leur partie principale et leur bord périphérique peut être pourvue, à la fois, d'au moins une gorge 19 et d'au moins un sillon 29.

## Revendications

1. Composant prothétique glénoïdien (10 ; 20),
comportant un corps (11 ; 21) d'appui pour une tête humérale prothétique (4) ou osseuse, lequel corps d'appui inclut, d'une part, une partie principale (16 ; 26) d'articulation avec la tête humérale et, d'autre part, un bord périphérique (17 ; 27), **caractérisé en ce que** la zone (18 ; 28) de liaison entre la partie principale (16 ; 26) et le bord périphérique (17 ; 27) est mécaniquement affaiblie pour, en service, rendre cette zone de liaison élastiquement déformable par rapport au reste du corps d'appui (11 ; 21) sous l'action de la tête humérale (4).

2. Composant suivant la revendication 1, **caractérisé en ce que** ladite zone de liaison (18 ; 28) est mécaniquement affaiblie suivant toute la périphérie du corps d'appui (11 ; 21).

3. Composant suivant la revendication 1, **caractérisé en ce que** ladite zone de liaison est mécaniquement affaiblie en une portion seulement ou en plusieurs portions distinctes suivant la périphérie du corps d'appui.

4. Composant suivant l'une quelconque des revendications précédentes, **caractérisé en ce que** ladite zone de liaison (18) est mécaniquement affaiblie par au moins une gorge (19) délimitée dans la face (13) du corps d'appui (11), opposée à celle (12) contre laquelle la tête humérale (4) s'appuie en service.

5. Composant suivant l'une quelconque des revendications précédentes, **caractérisé en ce que** ladite zone de liaison (28) est mécaniquement affaiblie par au moins un sillon (29) délimité dans la face (22) du corps d'appui (21), contre laquelle la tête humérale (4) s'appuie en service.

6. Composant suivant l'une quelconque des revendications précédentes, **caractérisé en ce que** le bord périphérique (17 ; 27) et ladite zone de liaison (18 ; 28) présentent, sur la face (12 ; 22) du corps d'appui (11 ; 21) contre laquelle la tête humérale (4) s'appuie en service, des surfaces respectivement convexe (S₁₇; S₂₇) et concave (S₁₈ ; S₂₈).

7. Composant suivant la revendication 6, **caractérisé en ce que** la partie principale (16 ; 26) présente, sur la face (12 ; 22) du corps d'appui (11 ; 21) contre laquelle la tête humérale (4) s'appuie en service, une surface concave (S₁₆ ; S₂₆) dont la courbure est moins grande que celle de la surface concave (S₁₈; S₂₈) de la zone de liaison (18 ; 28).

8. Composant suivant l'une quelconque des revendications précédentes, **caractérisé en ce que** le corps d'appui (11 ; 21) présente, à l'opposé de sa face (12 ; 22) contre laquelle la tête humérale (4) s'appuie en service, une face (13 ; 23) adaptée pour être plaquée contre et solidarisée à l'os d'une glène (G).

9. Composant suivant l'une quelconque des revendications précédentes, **caractérisé en ce que** le corps d'appui (11 ; 21) est monobloc.

10. Jeu d'au moins deux composants prophétiques glénoïdiens (10 ; 20), **caractérisé en ce que** chaque composant glénoïdien (10 ; 20) est conforme à l'une quelconque des revendications précédentes, et **en ce que**, suivant au moins une même direction passant par deux portions de la zone de liaison (18 ; 28) des composants, diamétralement opposées par rapport à la région centrale de la partie principale (16 ; 26) des composants, les corps d'appui (11 ; 21) de ces composants présentent une dimension ayant des valeurs différentes entre eux.

## Claims

1. A prosthetic glenoid component (10; 20), comprising a supporting member (11; 21) for a prosthetic or bone humeral head (4), which supporting member includes, on the one hand, a main part (16; 26) for articulation with the humeral head and, on the other hand, a peripheral rim (17; 27), **characterised in that** the connecting zone (18; 28) between the main part (16; 26) and the peripheral rim (17; 27) is mechanically weakened so as, when in service, to make said connecting zone resiliently deformable relative to the rest of the supporting member (11; 21) under the action of the humeral head (4).

2. A component according to claim 1, **characterised in that** said connecting zone (18; 28) is mechanically weakened over the entire periphery of the supporting member (11; 21).

3. A component according to claim 1, **characterised in that** said connecting zone is mechanically weakened in just one portion or in a plurality of separate portions over the periphery of the supporting member.

4. A component according to any one of the preceding claims, **characterised in that** said connecting zone (18) is mechanically weakened by at least one groove (19) defined in the opposite face (13) of the supporting member (11) from that (12) against which the humeral head (4) bears when in service.

5. A component according to any one of the preceding claims, **characterised in that** said connecting zone (28) is mechanically weakened by at least one notch (29) defined in the face (22) of the supporting member (21) against which the humeral head (4) bears when in service.

6. A component according to any one of the preceding claims, **characterised in that** the peripheral rim (17; 27) and said connecting zone (18; 28) have respectively convex (S₁₇; S₂₇) and concave (S₁₈; S₂₈) surfaces on the face (12; 22) of the supporting member (11; 21) against which the humeral head (4) bears when in service.

7. A component according to claim 6, **characterised in that** the main part (16; 26) has, on the face (12; 22) of the supporting member (11; 21) against which the humeral head (4) bears when in service, a concave surface (S₁₆; S₂₆) with curvature of lesser magnitude than that of the concave surface (S₁₈; S₂₈) of the connecting zone (18; 28).

8. A component according to any one of the preceding claims, **characterised in that** the supporting body (11; 21) has, on the opposite side from the face (12; 22) thereof against which the humeral head (4) bears when in service, a face (13; 23) suited to being fitted against and firmly connected to the bone of a glenoid (G).

9. A component according to any one of the preceding claims, **characterised in that** the supporting member (11; 21) is in one piece.

10. A set of at least two prosthetic glenoid components (10; 20), **characterised in that** each glenoid component (10; 20) is in accordance with any one of the preceding claims, and **in that**, in at least one and the same direction passing through two portions of the connecting zone (18; 28) of the components, diametrically opposite relative to the central region of the main part (16; 26) of the components, the supporting members (11; 21) of these components are of sizes which differ from one another.

## Patentansprüche

1. Prothetische Glenoidkomponente (10; 20), einen Stützkörper (11; 21) für einen prothetischen oder knöchernen Humeruskopf (4) umfassend, wobei dieser Stützkörper einerseits einen Hauptteil (16; 26) für eine Gelenkverbindung mit dem Humeruskopf und andererseits einen Umfangsrand (17; 27) einschließt, **dadurch gekennzeichnet, dass** die Verbindungszone (18; 28) zwischen dem Hauptteil (16; 26) und dem Umfangsrand (17; 27) mechanisch geschwächt ist, um im Einsatz diese Verbindungszone in Bezug auf den Rest des Stützkörpers (11; 21) unter der Wirkung des Humeruskopfes (4) elastisch deformierbar zu machen.

2. Komponente nach Anspruch 1, **dadurch gekennzeichnet, dass** die Verbindungszone (18; 28) gemäß dem gesamten Umfang des Stützkörpers (11; 21) mechanisch geschwächt ist.

3. Komponente nach Anspruch 1, **dadurch gekennzeichnet, dass** die Verbindungszone nur an einem Bereich oder an mehreren unterschiedlichen Bereichen gemäß dem Umfang des Stützkörpers mechanisch geschwächt ist.

4. Komponente nach einem der vorhergehenden Ansprüche, **dadurch gekennzeichnet, dass** die Verbindungszone (18) durch mindestens eine begrenzte Nut (19) in der Seite (13) des Stützkörpers (11), entgegengesetzt zu der (12), gegen die sich der Humeruskopf (4) im Einsatz abstützt, mechanisch geschwächt ist.

5. Komponente nach einem beliebigen der vorhergehenden Ansprüche, **dadurch gekennzeichnet, dass** die Verbindungszone (28) durch mindestens eine begrenzte Rille (29) in der Seite (22) des Stützkörpers (21), gegen die sich der Humeruskopf (4) im Einsatz abstützt, mechanisch geschwächt ist.

6. Komponente nach einem beliebigen der vorhergehenden Ansprüche, **dadurch gekennzeichnet, dass** der Umfangsrand (17; 27) und die Verbindungszone (18; 28) auf der Seite (12; 22) des Stützkörpers (11; 21) gegen die sich der Humeruskopf (4) im Einsatz abstützt, jeweils eine konvexe (S₁₇; S₂₇) und eine konkave Fläche (S₁₈; S₂₈) aufweisen.

7. Komponente nach Anspruch 6, **dadurch gekennzeichnet, dass** der Hauptteil (16; 26) auf der Seite (12; 22) des Stützkörpers, gegen die sich der Humeruskopf (4) im Einsatz abstützt, eine konkave Fläche (S₁₆; S₂₆) aufweist, deren Krümmung weniger groß als die der konkaven Fläche (S₁₈; S₂₈) der Verbindungszone (18; 28) ist.

8. Komponente nach einem beliebigen der vorhergehenden Ansprüche, **dadurch gekennzeichnet, dass** der Stützkörper (11; 21) entgegengesetzt zu seiner Seite (12; 22), gegen die sich der Humeruskopf (4) im Einsatz abstützt, eine Fläche (13; 23) aufweist, die angepasst ist, um gegen den Knochen einer Gelenkpfanne (G) gedrückt zu werden und mit ihm verbunden zu werden.

9. Komponente nach einem beliebigen der vorhergehenden Ansprüche, **dadurch gekennzeichnet, dass** der Stützkörper (11; 21) aus einem Stück besteht.

10. Satz von mindestens zwei prothetischen Glenoidkomponenten (10; 20), **dadurch gekennzeichnet, dass** jede Glenoidkomponente (10; 20) einem beliebigen der vorhergehenden Ansprüche entspricht und dass gemäß mindestens einer selben Richtung, die durch zwei Bereiche der Verbindungszone (18; 28) der Komponenten hindurchgeht, die in Bezug auf den Mittelbereich des Hauptteils (16; 26) der Komponenten diametral gegenüberliegen, die Stützkörper (11; 21) dieser Komponenten eine Abmessung aufweisen, die unterschiedliche Werte zueinander haben.
